(19)

Europäisches
Patentamt
European
Patent Office
Office européen
des brevets

(11)     **EP 4 513 189 A1**

(12)                 **EUROPEAN PATENT APPLICATION**
                     published in accordance with Art. 153(4) EPC

(43) Date of publication:
**26.02.2025  Bulletin 2025/09**

(21) Application number: **23792199.4**

(22) Date of filing: **20.04.2023**

(51) International Patent Classification (IPC):
**G01N 33/574** *(2006.01)*     **C07K 16/28** *(2006.01)*

(52) Cooperative Patent Classification (CPC):
**C07K 16/28; C12Q 1/6886; G01N 33/574**

(86) International application number:
**PCT/KR2023/005374**

(87) International publication number:
**WO 2023/204625 (26.10.2023 Gazette 2023/43)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **20.04.2022   KR 20220049175**

(71) Applicants:
• **Abion Inc.**
  **Seoul 08394 (KR)**
• **Samsung Life Public Welfare Foundation
  Seoul 140-893 (KR)**

(72) Inventors:
• **CHOI, Jun Young
  Gwangmyeong-si Gyeonggi-do 14315 (KR)**

• **KIM, Na Young
  Anyang-si Gyeonggi-do 13990 (KR)**
• **PARK, Kyung Eui
  Seoul 08028 (KR)**
• **LEE, Young Jin
  Seoul 03464 (KR)**
• **CHOI, Yoon La
  Seoul 06351 (KR)**

(74) Representative: **Schwarz & Partner Patentanwälte
GmbH
Patentanwälte
Wipplingerstraße 30
1010 Wien (AT)**

Remarks:
The complete document including Reference
Table(s) and the Sequence Listing(s) can be
downloaded from the EPO website

(54)   **METHOD FOR PREDICTING PROGNOSIS OF BREAST CANCER PATIENT USING CTC
       EXPRESSING C-MET**

(57)      The present invention relates to a method for
predicting the prognosis of a breast cancer patient using
c-Met enriched CTCs, and more specifically, to a method
for predicting the prognosis of breast cancer by isolating
and counting c-Met enriched CTCs from a breast cancer
patient, and to a composition for predicting the prognosis
of breast cancer comprising an agent that specifically
binds to c-Met enriched CTCs. The method and composition of the present invention can be usefully employed
to accurately predict the prognosis of a breast cancer
patient without the need for a tissue biopsy.

FIG. 1a

HR+ mBC (n=93)

Low group : median PFS 5.5 months (95% CI, 2.8-NA)
High group : median PFS 9.9 months (95% CI, 7.4-NA)

**EP 4 513 189 A1**

FIG. 1b

**HR+ mBC (n=93)**

Low group : median PFS 9.4 months (95% CI, 5.8-NA)
High group : median PFS 7.5 months (95% CI, 2.8-NA)

**Description**

**TECHNICAL FIELD**

This application claims priority to Korean Patent Application No. 10-2022-0049175, filed on April 20, 2022, the entire disclosure of which is incorporated herein by reference.

The present invention relates to a method for predicting the prognosis of a breast cancer patient using c-Met enriched circulating tumor cells (CTCs). More specifically, the invention provides a method for predicting the prognosis of breast cancer by isolating and c-Met enriched CTCs from a breast cancer patient.

**BACKGROUND OF THE INVENTION**

Breast cancer is the most common cancer among women and the second leading cause of cancer-related deaths. Risk factors for breast cancer include race, age, and mutations in tumor suppressor genes such as BRCA-1, BRCA-2, and p53. Additionally, alcohol consumption, highfat diets, lack of exercise, exogenous postmenopausal hormones, and ionizing radiation also increase the risk of developing breast cancer. Breast cancer is classified into four subtypes based on the expression status of hormone receptors (estrogen receptor or progesterone receptor) and HER2 (human epidermal growth factor receptor 2): Luminal A, Luminal B, HER2-enriched, and triple-negative breast cancer (TNBC).

Current treatment methods for breast cancer often require additional adjuvant therapies such as chemotherapy, hormone therapy, targeted therapy, or radiation therapy after surgery to reduce the risk of recurrence. The characteristics of breast cancer vary depending on the status of major breast cancer receptors. Therefore, tissue biopsies are conducted to establish the basis for subsequent treatment methods by evaluating the presence and overexpression of hormone receptors (estrogen receptor [ER] and progesterone receptors[PR]), the status of metastasis, and the involvement of lymph node (positive or negative). However, tissue-based examinations reflect the biomarker status at the time of tissue collection and involve invasive methods (e.g., biopsy, resection), which pose significant risks and burdens to patients.

Meanwhile, circulating tumor cells (CTCs) are tumor cells found in the peripheral blood of patients with malignant tumors. CTCs play a crucial role in the metastasis process, making them highly significant in cancer research and diagnosis. However, due to the extremely low number of circulating tumor cells present in peripheral blood, a detection system with high sensitivity capable of identifying a few dozen tumor cells among millions of normal blood cells is required.

Methods for isolating CTCs can be broadly categorized into physical isolation methods, which utilize the physical properties of CTCs such as size, deformability, density, or charge, and biological isolation methods, which rely on markers detected by antibodies, antibody-coated beads, magnetic beads, magnetic materials, or aptamers. The only FDA-approved device employs a biological isolation method, using EpCAM (epithelial cell adhesion molecule) antibodies to isolate cells and immunostaining to identify cancer cells. Consequently, various studies have been conducted using EpCAM-enriched CTCs. However, a limitation exists in that EpCAM expression decreases during epithelial-mesenchymal transition (EMT) associated with metastasis, making it difficult to detect these cells.

Therefore, there is a need for a new method that utilizes CTCs to predict breast cancer prognosis while maintaining the accuracy of tissue-based methods, thereby reducing the risks and burdens on patients.

**PRIOR ART DOCUMENTS**

**PATENT DOCUMENTS**

(Patent Document 1) Korean Patent Publication No. 10-2020-0069822
(Patent Document 2) Korean Patent Publication No. 10-2020-0069839

**SUMMARY OF INVENTION**

**PROBLEM TO BE SOLVED**

The inventors of the present invention have conducted extensive research to develop a method for accurately predicting the prognosis of breast cancer patients. As a result, we have discovered for the first time that isolating c-Met enriched circulating tumor cells (CTCs) from the blood of breast cancer patients and counting them, the prognosis of breast cancer patients can be accurately predicted.

Therefore, an object of the present invention is to provide a method for providing information necessary for predicting the prognosis of a breast cancer patient, comprising the steps of:

(a) isolating c-Met enriched circulating tumor cells (CTCs) from a biological sample obtained from a breast cancer patient;
(b) counting the CTCs isolated in step (a); and
(c) predicting the prognosis of breast cancer based on the count obtained in step (b), wherein a lower count indicates a better prognosis.

Another object of the present invention is to provide a method for providing information necessary for predicting the prognosis of a breast cancer patient, comprising the steps of:

(a) isolating cell-free DNA (cfDNA) from a biological sample obtained from a breast cancer patient;
(b) calculating the concentration of cfDNA or the mutation index of the ESR1 gene in the cfDNA isolated in step (a); and
(c) predicting the prognosis of breast cancer based on the value calculated in step (b), wherein a lower value indicates a better prognosis.

Another object of the present invention is to provide a composition for predicting the prognosis of breast cancer, comprising an agent that specifically binds to c-Met enriched CTCs.

Additionally, another object of the present invention is to provide a composition for predicting the prognosis of breast cancer, consisting of an agent that specifically binds to c-Met enriched CTCs.

Furthermore, another object of the present invention is to provide a composition for predicting the prognosis of breast cancer, consisting essentially of an agent that specifically binds to c-Met enriched CTCs.

Another object of the present invention is to provide the use of an agent that specifically binds to c-Met enriched CTCs in the manufacturing a composition for predicting the prognosis of breast cancer.

## MEANS FOR SOLVING THE PROBLEM

To achieve the above objectives, the present invention provides a method for providing information necessary for predicting the prognosis of a breast cancer patient, comprising the steps of:

(a) isolating c-Met enriched circulating tumor cells (CTCs) from a biological sample obtained from a breast cancer patient;
(b) counting the CTCs isolated in step (a); and
(c) predicting the prognosis of breast cancer based on the count obtained in step (b), wherein a lower count indicates a better prognosis.

To achieve another objective of the present invention, the present invention provides a method for providing information necessary for predicting the prognosis of a breast cancer patient, comprising the steps of:

(a) isolating cell-free DNA (cfDNA) from a biological sample obtained from a breast cancer patient;
(b) calculating the concentration of cfDNA or the mutation index of the ESR1 gene in the cfDNA isolated in step (a); and
(c) predicting the prognosis of breast cancer based on the value calculated in step (b), wherein a lower value indicates a better prognosis.

To achieve another objective of the present invention, the present invention provides a composition for predicting the prognosis of breast cancer, comprising an agent that specifically binds to c-Met enriched CTCs.

Additionally, to achieve another objective of the present invention, the present invention provides a composition for predicting the prognosis of breast cancer, consisting of an agent that specifically binds to c-Met enriched CTCs.

Furthermore, to achieve another objective of the present invention, the present invention provides a composition for predicting the prognosis of breast cancer, consisting essentially of an agent that specifically binds to c-Met enriched CTCs.

To achieve another objective of the present invention, the present invention provides the use of an agent that specifically binds to c-Met enriched CTCs for manufacturing a composition for predicting the prognosis of breast cancer.

Hereinafter, the present invention will be described in detail.

The present invention provides a method for providing information necessary for predicting the prognosis of a breast cancer patient, comprising the steps of:

(a) isolating c-Met enriched CTCs from a biological sample obtained from a breast cancer patient.

(b) counting the CTCs isolated in step (a); and

(c) predicting the prognosis of breast cancer based on the count obtained in step (b), wherein a lower count indicates a better prognosis.

Breast cancer is classified based on genetic and molecular biological characteristics (Table 1). It has been reported that the outcomes and prognosis vary depending on the subtype, which is used as an indicator for selecting surgical methods or chemotherapy.

Table 1

| Classification of Molecular Biological Subtypes of Breast Cancer | | |
|---|---|---|
| Subtype | Characteristics | Incidence(%) |
| Luminal A (HR+/HER2-) | * ER positive and/or PR positive<br>* HER2 negative<br>* low Ki67 expression | 30~70 |
| Luminal B (HR+/HER2+) | * ER positive and/or PR positive<br>* HER2 positive (or HER 2 negative with high Ki67 expression) | 10~20 |
| HER2 (HR-/HER2+) | * ER negative<br>* PR negative<br>* HER2 positive | 5~15 |
| triple negative | * ER negative<br>* PR negative<br>* HER2 negative | 15~20 |

Among these, the breast cancer in the present invention is preferably breast cancer that is estrogen receptor (ER) and/or progesterone receptor (PR) positive (HR+) and HER2 negative (HER2-), or breast cancer that is ER and/or PR positive (HR+) and HER2 positive (HER2+), and may be metastatic or non-metastatic breast cancer, but is not limited thereto.

In the present invention, "prognosis" refers to the course of the disease during or after breast cancer treatment, preferably the course of the disease after treatment, but is not limited thereto. Additionally, the "course of the disease" includes concepts such as complete remission, recurrence, metastasis, metastatic recurrence, disease-free survival, distant metastasis-free survival, or progression-free survival, preferably progression-free survival, but is not limited thereto.

Meanwhile, "progression-free survival (PFS)" refers to the time during or after treatment for a disease like cancer when the patient lives with the disease but it does not worsen. PFS is a commonly used measure to evaluate the effectiveness of cancer treatment, and the time interval from the start of treatment to the progression of the disease is a representative definition of progression-free survival, serving as a clinical benefit measure of the treatment.

Hereinafter, each step of the method for providing information necessary for predicting the prognosis of breast cancer according to the present invention will be described in more detail.

(a) isolating c-Met enriched circulating tumor cells (CTCs) from a biological sample obtained from a breast cancer patient;

In the present invention, the biological sample may be blood, plasma, serum, whole blood, isolated blood cells, bone marrow fluid, lymph fluid, saliva, urine, mucosal fluid, amniotic fluid, or a combination thereof obtained from a breast cancer patient, preferably blood, plasma, serum, whole blood, or a combination thereof obtained from a breast cancer patient, and most preferably blood obtained from a breast cancer patient, but is not limited thereto.

In the present invention, c-Met enriched circulating tumor cells (CTCs) refer to CTCs in which c-Met is expressed and widely distributed on the cell surface.

The "c-Met" refers to a representative receptor tyrosine kinase (RTK) present on the cell surface, which, when bound to its ligand HGF/SF (Hepatocyte Growth Factor/Scattering Factor), promotes intracellular signal transduction, thereby promoting cell growth and being widely involved in cancer occurrence, metastasis, cell migration, invasion, and angiogenesis in many types of cancer cells. As the name of the ligand suggests, c-Met signaling through HGF/SF weakens cell-cell contact in almost all types of epithelial tumors, causing scattering, which is a representative early stage of cancer metastasis.

In the present invention, the step of isolating c-Met enriched CTCs can be performed according to conventional methods known in the art. Methods for isolating CTCs can be broadly divided into physical isolation methods that utilize the physical properties of CTCs such as size, deformability, density, or charge, and biological isolation methods that rely on markers detected by other substances such as antibodies, beads, magnetic beads, magnetic materials, or aptamers.

In the method according to the present invention, when using a physical isolation method for CTCs, c-Met enriched CTCs necessary for the method according to the present invention can be isolated by performing a process of staining c-Met to distinguish c-Met enriched CTCs from the isolated CTCs.

In the method according to the present invention, when using a biological isolation method for CTCs, the antibody is preferably an antibody that specifically binds to c-Met. The 'magnetic material to which the antibody is attached' is not limited thereto but may include a substance containing a magnetic metal or magnetic metal oxide, preferably Co, Mn, Fe, Ni, Gd, $MM'_2O_4$, and $MxOy$ (where M or M' represents Co, Fe, Ni, Mn, Zn, Gd, or Cr, and x and y represent integers).

In the method according to the present invention, it is apparent to a person skilled in the art that the effect is the same regardless of which method of isolating CTCs is used.

(b) counting the CTCs isolated in step (a);
In the present invention, the step of counting the CTCs can be performed by methods known in the art. Methods for counting CTCs include, but are not limited to, molecular analysis methods based on nucleic acid analysis such as classical immunocytochemistry, laser scanning cytometry, reverse transcription quantitative polymerase chain reaction, multiplex reverse transcription polymerase chain reaction and etc, and protein-based analysis methods such as EPISPOT assay (Epithelial Immuno-SPOT assay).
(c) predicting the prognosis of breast cancer based on the count obtained in step (b), wherein a lower count indicates a better prognosis;

According to one embodiment of the present invention, it was confirmed that the count of c-Met enriched CTCs is highly correlated with the prognosis of breast cancer patients. Therefore, the lower the count of c-Met enriched CTCs, the better the prognosis of the breast cancer patient, and conversely, the higher the count of c-Met enriched CTCs, the worse the prognosis of the breast cancer patient.

In the present invention, the term "high" or "low" in the count of c-Met enriched CTCs means that when compared to the count of c-Met enriched CTCs isolated from a biological sample obtained from a normal control sample, the count of c-Met enriched CTCs isolated from a biological sample obtained from a breast cancer patient is higher or lower, respectively. Alternatively, when the count of c-Met enriched CTCs isolated from a biological sample obtained from a breast cancer patient is below a certain cut-off value calculated using statistical methods, it is judged to have a good prognosis, and when it is above a certain cut-off value, it is judged to have a poor prognosis. The cut-off value can be easily calculated using various cut-off calculation methods widely known in the art, such as the maxstat package or Youden Index, and a person skilled in the art can easily apply the cut-off value to the prognosis prediction method according to the present invention.

In the present invention, "good prognosis" refers to a low probability of recurrence, metastasis, or metastatic recurrence, and high disease-free survival, distant metastasis-free survival, or progression-free survival, preferably high progression-free survival. "Poor prognosis" refers to a high probability of recurrence, metastasis, or metastatic recurrence, and low disease-free survival, distant metastasis-free survival, or progression-free survival, preferably low progression-free survival.

Additionally, the present invention provides a method for providing information necessary for predicting the prognosis of a breast cancer patient, comprising the steps of:

(a) isolating cell-free DNA (cfDNA) from a biological sample obtained from a breast cancer patient.
(b) calculating the concentration of cfDNA or the mutation index of the ESR1 gene in the cfDNA isolated in step (a); and
(c) predicting the prognosis of breast cancer based on the value calculated in step (b), wherein a lower value indicates a better prognosis.

The terms used in the method according to the present invention are as described above, and hereinafter, terms used only in the method will be explained.

In the present invention, cell-free DNA (cfDNA) refers to DNA fragments that are not present in the cell nucleus but are floating in the blood. It refers to DNA derived from cells present in the plasma. The cfDNA typically has a double helix structure and often has a coiled-coil structure. The cfDNA may be derived from tumor cells. Additionally, cfDNA derived from tumor cells can be found in biological samples such as blood, plasma, or urine obtained from cancer patients. The step of isolating cfDNA in the present invention can be performed according to conventional methods known in the art, and it is apparent to a person skilled in the art that the effect is the same regardless of which known method for isolating cfDNA is used.

In the method according to the present invention, the concentration of cfDNA can be calculated by the following formula:

$$\text{cfDNA concentrations/mL plasma}$$
$$= \text{IC copies/well} \times \frac{\text{total DNA elution volume}}{\text{DNA loading volume/well}} \times \frac{1}{\text{plasma volume (mL)}}$$

In the present invention, the estrogen receptor (Estrogen Receptor 1, ESR1) initiates ligand-activated transcription, and cyclin D1 (CCND1), a member of the cyclin family, and CDK-activating kinase 7 (CDK7) are key regulators of cell cycle progression and cell development. When a quiescent cell enters the cell cycle, the expression of CCND1 is induced by the binding of estrogen to the estrogen receptor (ER), and after CCND1 binds to CDK4 and CDK6 and enters the cell nucleus, it is phosphorylated by CDK7, leading to the phosphorylation of protein substrates. CDK7 functions as a CDK-activating kinase (CAK) in complex with cyclin H, and these induce the activating phosphorylation of various CDKs, thereby regulating the cell cycle.

In the present invention, the mutation of the ESR1 gene refers to a hot-spot mutation of ESR1, which occurs at any one of the amino acids selected from the group consisting of E380, S463, V534, L536, Y537, and D538.

In the present invention, the mutation index of the ESR1 gene can be calculated by the following formula:

$$\text{Mutation Index} = \frac{\text{Mutant copies of PIK3CA or ESR1}}{\text{Total copies of PIK3CA or ESR1}} \times 100\%$$

According to one embodiment of the present invention, it was confirmed that the concentration of cfDNA or the mutation index of the ESR1 gene in cfDNA is highly correlated with the prognosis of breast cancer patients. Therefore, the lower the concentration of cfDNA or the mutation index of the ESR1 gene, the better the prognosis of the breast cancer patient, and conversely, the higher the concentration of cfDNA or the mutation index of the ESR1 gene, the worse the prognosis of the breast cancer patient.

In the present invention, the term "high" or "low" in the concentration of cfDNA or the mutation index of the ESR1 gene refers to that when compared to the concentration of cfDNA or the mutation index of the ESR1 gene isolated from a biological sample obtained from a normal control sample, the concentration of cfDNA or the mutation index of the ESR1 gene isolated from a biological sample obtained from a breast cancer patient is higher or lower, respectively. Alternatively, when the concentration of cfDNA or the mutation index of the ESR1 gene isolated from a biological sample obtained from a breast cancer patient is below a certain cut-off value calculated using statistical methods, it is judged to have a good prognosis, and when it is above a certain cut-off value, it is judged to have a poor prognosis. The cut-off value can be easily calculated using various cut-off calculation methods widely known in the art, such as the maxstat package or Youden Index, and a person skilled in the art can easily apply the cut-off value to the prognosis prediction method according to the present invention.

Additionally, the present invention provides a composition for predicting the prognosis of breast cancer, comprising an agent that specifically binds to c-Met enriched circulating tumor cells (CTCs).

In the present invention, the agent that specifically binds to c-Met enriched circulating tumor cells (CTCs) may be selected from the group consisting of an antibody, aptamer, DNA, RNA, protein, or polypeptide, preferably an antibody or aptamer, and most preferably an antibody, but is not limited thereto.

In one embodiment of the present invention, the antibody may be an antibody comprising a light chain variable region (VL) including a complementarity-determining region (CDR) L1 comprising the amino acid sequence of SEQ ID NO: 1, a CDR L2 comprising the amino acid sequence of SEQ ID NO: 2, and a CDR L3 comprising the amino acid sequence of SEQ ID NO: 3, and a heavy chain variable region (VH) including a CDR H1 comprising the amino acid sequence of SEQ ID NO: 4, a CDR H2 comprising the amino acid sequence of SEQ ID NO: 5, and a CDR H3 comprising the amino acid sequence of SEQ ID NO: 6, and specifically binding to the c-Met protein; or a fragment thereof.

In another embodiment of the present invention, the antibody may be an antibody comprising a light chain variable region (VL) including a complementarity-determining region (CDR) L1 comprising the amino acid sequence of SEQ ID NO: 7, a CDR L2 comprising the amino acid sequence of SEQ ID NO: 8, and a CDR L3 comprising the amino acid sequence of SEQ ID NO: 9, and a heavy chain variable region (VH) including a CDR H1 comprising the amino acid sequence of SEQ ID NO: 10, a CDR H2 comprising the amino acid sequence of SEQ ID NO: 11, and a CDR H3 comprising the amino acid sequence of SEQ ID NO: 12, and specifically binding to the c-Met protein; or a fragment thereof.

In the present invention, 'antibody', 'anti-c-Met antibody', 'humanized anti-c-Met antibody', and 'modified humanized anti-c-Met antibody' are used in the broadest sense in the present invention, specifically including monoclonal antibodies (including full-length monoclonal antibodies), polyclonal antibodies, multispecific antibodies (e.g., bispecific antibodies), and antibody fragments (e.g., variable regions and other parts of the antibody that exhibit the desired biological activity, such as binding to c-Met).

The antibody of the present invention includes both monoclonal and polyclonal antibodies, with specific amino acid sequences in the light and heavy chain CDRs that allow selective binding to c-Met, and preferably, it may be a monoclonal antibody. Additionally, the antibody of the present invention includes chimeric antibodies, humanized antibodies, and human antibodies, and preferably, it may be a human antibody.

The monoclonal antibody of the present invention represents an antibody obtained from a substantially homogeneous population of antibodies, meaning that individual antibodies in the population are identical except for possible naturally occurring mutations that may be present in minor amounts. Monoclonal antibodies bind very specifically to a single antigen epitope.

In the present invention, the term 'monoclonal' refers to the characteristic of the antibody being obtained from a substantially homogeneous population and does not necessarily imply that the antibody must be produced by a specific method. For example, the monoclonal antibody of the present invention can be produced by the hybridoma method first disclosed in the art or by recombinant DNA methods (see U.S. Patent No. 4,816,567). Additionally, it can be isolated from a phage antibody library using techniques known in the art.

The antibody of the present invention specifically includes chimeric antibodies, where parts of the heavy and/or light chains originate from or are identical or homologous to corresponding sequences from a particular species or antibody, while the remaining parts originate from or are identical or homologous to corresponding sequences from another species or antibody, as long as the antibody of the present invention exhibits the desired biological activity (e.g., selective binding to NRS) (see U.S. Patent No. 4,816,567).

Humanized antibodies are antibodies that include sequences from both human and non-human (e.g., mouse, rat) antibodies, generally where the parts other than the epitope-binding regions (CDRs) are from human antibodies, and the epitope-binding regions (CDRs) may include sequences derived from non-human sources. Fully human antibodies refer to antibodies that include only human immunoglobulin protein sequences and can be produced in mice, mouse cells, or hybridomas derived from mouse cells, or by phage display methods.

Naturally occurring antibodies produced in vivo are typically heterotetrameric glycoproteins of about 150,000 daltons, composed of two identical light chains (L) and two identical heavy chains (H). Each light chain is connected to a heavy chain by a single covalent disulfide bond, but the number of disulfide bonds varies among different immunoglobulin isotypes. Each heavy and light chain also has regularly spaced intrachain disulfide bridges. Each heavy chain has a variable domain (VH) at one end followed by several constant domains. Each light chain has a variable domain (VL) at one end and a constant domain at the other end; the constant domain of the light chain aligns with the first constant domain of the heavy chain, and the variable domain of the light chain aligns with the variable domain of the heavy chain. Specific amino acid residues are believed to form the interface between the variable domains of the light and heavy chains. The "variable region" or "variable domain" of an antibody refers to the amino-terminal domains of the heavy or light chains. The variable region of the heavy chain is designated "VH," and the variable region of the light chain is designated "VL." These domains are generally the most variable parts of the antibody and include the antigen-binding sites.

In the present invention, 'hypervariable' refers to the fact that certain sequences within the variable regions vary widely among antibodies and include residues directly related to the binding and specificity of each specific antibody to its specific antigenic determinants. Hypervariability in both the light and heavy chain variable regions is concentrated in three segments known as complementarity-determining regions (CDRs) or hypervariable loops (HVLs). While CDRs are defined by sequence comparison known in the art, HVLs are structurally defined based on the three-dimensional structure of the variable regions as known in the art.

The three CDRs within each of the heavy and light chains are separated by framework regions (FRs), which tend to include less variable sequences. From the amino terminus to the carboxy terminus of the variable regions of the heavy and light chains, the FRs and CDRs are arranged in the following order: FR1, CDR1, FR2, CDR2, FR3, CDR3, and FR4. The large β-sheet arrangement of the FRs brings the CDRs within each chain close to each other and to the CDRs from the other chain. The resulting structure contributes to the antigen-binding site, although not all CDR residues need to be directly involved in antigen binding.

The fragment of the present invention is characterized by being selected from the group consisting of diabody, Fab, Fab', F(ab)2, F(ab')2, Fv, and scFv.

In the present invention, an antibody fragment refers to a fragment of an antibody that retains the antigen-specific binding ability of the whole antibody, and preferably, the fragment retains at least 20%, 50%, 70%, 80%, 90%, 95%, or 100% or more of the parent antibody's affinity for human-derived c-Met protein. Specifically, it may be in the form of Fab, F(ab)2, Fab', F(ab')2, Fv, diabody, or scFv.

Fab (fragment antigen-binding) is an antigen-binding fragment of an antibody, consisting of one variable domain and one constant domain of each of the heavy and light chains. F(ab')2 is a fragment generated by pepsin digestion of an antibody, with two Fab fragments connected by disulfide bonds at the hinge region of the heavy chains. F(ab') is a monomeric antibody fragment with a hinge region added to the Fab fragment obtained by reducing the disulfide bonds of the F(ab')2 fragment. Fv (variable fragment) is an antibody fragment consisting only of the variable regions of the heavy and light chains. scFv (single chain variable fragment) is a recombinant antibody fragment in which the variable regions of the heavy chain (VH) and the light chain (VL) are connected by a flexible peptide linker. Diabody is a fragment in which the VH and VL of scFv are connected by a very short linker, preventing them from binding to each other, and instead, they bind to the VL and VH of another scFv of the same type, forming a dimer.

For the purposes of the present invention, an antibody fragment is not limited by its structure or form as long as it retains the binding specificity for human-derived c-Met protein, but preferably, it may be scFv. The scFv according to the present invention has the CDR composition or the VH and VL composition specific to the human-derived c-Met protein described above, and the sequence is not particularly limited as long as the C-terminus of VH and the N-terminus of VL are connected by a linker. The linker is not particularly limited as long as it is known in the art as a linker applicable to scFv.

The antibody or fragment of the present invention may include conservative amino acid substitutions (referred to as conservative variants of the antibody) that do not substantially alter its biological activity.

Additionally, the aforementioned antibody or fragment of the present invention may be conjugated with enzymes, fluorescent substances, radioactive substances, proteins, etc., but is not limited thereto. Methods for conjugating such substances to antibodies are well known in the art.

The antibody of the present invention may be derived from any animal, including mammals and birds, including humans. Preferably, the antibody may be an antibody from humans, mice, donkeys, sheep, rabbits, goats, guinea pigs, camels, horses, or chickens, and most preferably, it may be from humans or mice.

Human antibodies are antibodies with the amino acid sequence of human immunoglobulins, including antibodies isolated from a human immunoglobulin library or antibodies isolated from animals that have been transgenically modified to express one or more human immunoglobulins and do not express endogenous immunoglobulins (see U.S. Patent No. 5,939,598).

The antibody of the present invention may be conjugated with enzymes, fluorescent substances, radioactive substances, proteins, etc., but is not limited thereto. Methods for conjugating such substances to antibodies are well known in the art.

The present invention provides the use of an agent that specifically binds to c-Met enriched circulating tumor cells (CTCs) for the preparation of a composition for predicting the prognosis of breast cancer.

In this specification, the term "comprising" is used in the same sense as "including" or "characterized by," and does not exclude additional components or steps not specifically mentioned in the composition or method according to the present invention. The term "consisting of" means excluding additional elements, steps, or components not specifically described. The term "consisting essentially of" means that the composition or method may include substances or steps that do not materially affect the basic characteristics of the described substances or steps.

## EFFECT OF THE INVENTION

Accordingly, the present invention provides a method for predicting the prognosis of breast cancer using c-Met enriched CTCs and a composition for predicting the prognosis of breast cancer comprising an agent that specifically binds to c-Met enriched CTCs. By using the method and composition of the present invention, it is possible to accurately predict the prognosis of a breast cancer patient without a tissue biopsy.

## BRIEF DESCRIPTION OF THE DRAWINGS

FIGS. 1a and 1b show survival curve analyses for progression-free survival based on c-Met enriched CTC count (FIG. 1a) and EpCAM enriched CTC count (FIG. 1b) in HR+ metastatic breast cancer patients.

FIGS. 2a and 2b show survival curve analyses for progression-free survival based on c-Met enriched CTC count (FIG. 2a) and EpCAM enriched CTC count (FIG. 2b) in HR+/HER2-metastatic breast cancer patients.

FIGS. 3a and 3b show survival curve analyses for progression-free survival based on c-Met enriched CTC count (FIG. 3a) and EpCAM enriched CTC count (FIG. 3b) in HR+/HER2+ metastatic breast cancer patients.

FIGS. 4a to 4f illustrate a schematic workflow of cfDNA analysis (FIG. 4a), the correlation between ESR1 copies/mL plasma and PIK3A copies/mL plasma (FIG. 4b), and Kaplan-Meier analysis results for PFS under various conditions, where FIG. 4c shows cfDNA concentration in HR+/HER2- mBC, FIG. 4d shows cfDNA concentration in HR+/HER2+ mBC, FIG. 4e shows ESR1 hotspot mutation in HR+/HER2- mBC, and FIG. 4f shows PIK3CA hotspot mutation in HR+/HER2- mBC.

FIGS. 5a to 5d show Kaplan-Meier survival analysis results based on CTC and plasma cfDNA concentrations in HR+/HER2- mBC, where FIGS. 5a and 5b show analyses of c-Met enriched CTC and cfDNA, and FIGS. 5c and 5d show analyses of EpCAM enriched CTC and cfDNA. Among these, FIGS. 5a and 5c illustrate the classification of HR+/HER2- mBC patients into four categories, and FIGS. 5b and 5d illustrate the classification of HR+/HER2- mBC patients into four categories.

FIG. 6 shows the results of Cox regression multivariate analysis in HR+HER2- mBC patient groups.

## DETAILED DESCRIPTION OF THE INVENTION

The present invention will be described in detail below. However, the following examples are merely illustrative of the present invention, and the scope of the present invention is not limited to these examples.

## Example 1. Isolation and Counting of CTCs in Breast Cancer Patients

### 1-1. Isolation of CTCs in Breast Cancer Patients

Blood samples of 10 mL were collected from 97 HR+ metastatic breast cancer patients who consented to participate in the study. Analysis was conducted on 93 patients, excluding 4 patients with RBC contamination. The samples were anonymized by assigning numbers in the order of collection. The collected blood was immediately placed in a cell-free DNA blood collection tube (treck, La Viasta, NE, USA) and mixed thoroughly with the anticoagulant by inverting the tube. The samples were stored at room temperature until the isolation of circulating tumor cells (CTCs). The samples were processed using the GenoCTC V2 and separation kit (Genobio Corp, Seoul, South Korea) to isolate EpCAM-expressing CTCs and c-MET-expressing CTCs through microfluidics and magnetic separation specific to certain proteins.

For the isolation, 4 mL of whole blood was mixed with a substance combining anti-c-MET antibody and magnetic beads, and another 4 mL of whole blood was mixed with a substance combining anti-EpCAM antibody and magnetic beads, and incubated at room temperature for 30 minutes. After the reaction with the antibody-bound magnetic beads, the samples were processed using the GenoCTC equipment according to the manufacturer's protocol to isolate the circulating tumor cells. After isolation, 2% BSA was added to the collected solution from the central line to achieve a final concentration of 0.1% BSA, followed by centrifugation at 100g for 5 minutes. The supernatant was removed with a pipette, and the remaining solution was mixed well and placed on a slide glass. The slide glass was then placed on a heat block set at 40°C to evaporate the solution.

Peripheral blood mononuclear cells (PBMCs) were isolated using Ficoll-paque according to the manual's instructions. After mixing PBS and whole blood in a 1:1 ratio, the mixture was carefully layered over Ficoll-paque in a conical tube and centrifuged at 400g for 30 minutes to obtain PBMCs, which were then washed with PBS and centrifuged at 100g for 5 minutes. The isolated PBMCs were also placed on a heat block set at 40°C to evaporate the solution.

### 1-2. Staining of Isolated CTCs

After the solution was evaporated, immunocytochemical staining was performed using the GenoCTC profiling kit (Genobio corp.). A solution for fixing and permeabilizing the cells attached to the slide was loaded and incubated at room temperature for 10 minutes. After washing with PBS, blocking was performed with 2% BSA at room temperature for 10 minutes. Fluorescently labeled anti-CK18 and anti-CD45 antibodies were then added and incubated at room temperature for 1 hour for staining. The reaction was protected from light using aluminum foil. After the reaction, the slides were washed with PBS to remove unbound antibodies, stained with DAPI for nuclear staining, and covered with a cover slide to fix the slides.

## Example 2. Analysis of Progression-Free Survival and Cox Proportional Hazards Model Based on CTC Count

The cells attached to the slides from Example 1 were counted using a fluorescence microscope. Among 97 patients with HR+ metastatic breast cancer, data from 93 patients, excluding 4 cases with RBC contamination during CTC isolation, were analyzed. Circulating tumor cells were defined as CK18+, CD45-, and DAPI+ cells. The sensitivity of CD45 fluorescence was adjusted based on slides with PBMCs and applied to slides with isolated circulating tumor cells. The circulating tumor cells were photographed, and the counting results were recorded.

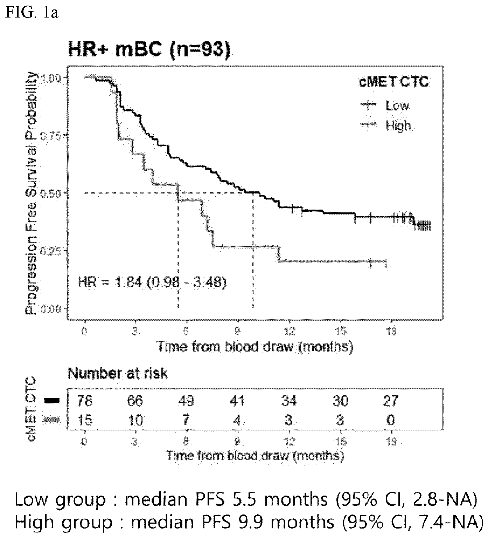

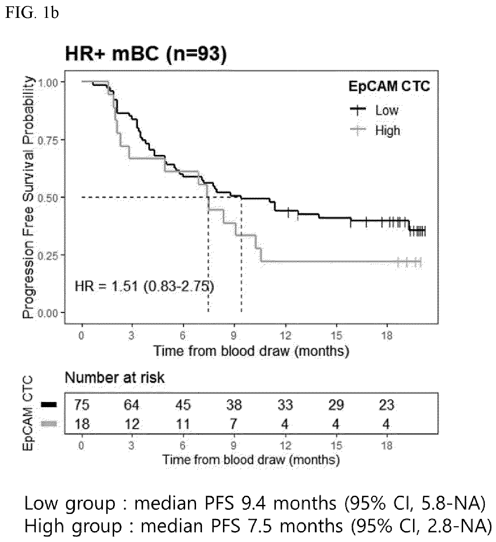

After patient recruitment was completed, clinical and pathological information and progression-free survival data were independently collected. Progression-free survival was defined based on radiological assessment according to RECIST v1.1, calculated from the date of blood collection to the occurrence of progression or death, whichever occurred first. Statistical analysis was performed using the R programming environment (ver. 1.4.1103), and the cut-off was calculated using the maxstat R package based on standardized log-rank statistics. The survival curve analysis and Cox proportional hazards model were used to analyze the association between c-Met-expressing CTCs, EpCAM-expressing CTCs, and other clinical and pathological information with progression. Statistical significance was set at $p\text{-value} < 0.05$ for analysis. The results are shown in Figures 1a and 1b and Table 2.

Table 2

| | | Univariate | | Multivariate | |
|---|---|---|---|---|---|
| | | HR (95% CI) | p value | HR (95% CI) | p value |
| HR+ (N=93) | Age (≥50) | 1.1 (0.64-1.8) | 0.77 | 1.3 (0.76-2.35) | 0.31 |
| | Visceral metastasis | 1.3 (0.76-2.2) | 0.34 | 1.2 (0.65-2.1) | 0.60 |
| | HER2 expression in primary tumor | 0.51 (0.28-0.93) | 0.029 | 1.4 (0.34-5.9) | 0.63 |
| | Hormone therapy at the time of blood collection | 1.1 (0.64-1.8) | 0.81 | 1.4 (0.30-6.8) | 0.66 |
| | Chemotherapy at the time of blood collection | 2.9 (1.7-5) | 0.0001 | 3.7 (0.74-19.0) | 0.11 |
| | HER2 targeted therapy at the time of blood collection | 0.33 (0.17-0.64) | 0.0009 | 0.41 (0.10-1.6) | 0.20 |
| | EpCAM+ CTC (≥4) | 1.5 (0.83-2.7) | 0.18 | 1.7 (0.87-3.1) | 0.12 |
| | **cMET+ CTC (≥3)** | **1.8 (0.98-3.5)** | **0.06** | **2.7 (1.2-6.02)** | **0.014** |

*HR, Hazard ratio; CI, confidence interval;*

As shown in Figures 1a and 1b, the count of c-Met-expressing CTCs in HR+ metastatic breast cancer patients was found to be a significant factor associated with progression-free survival (Figure 1a). In contrast, EpCAM-overexpressing CTCs did not show a statistically significant association with progression-free survival (Figure 1b).

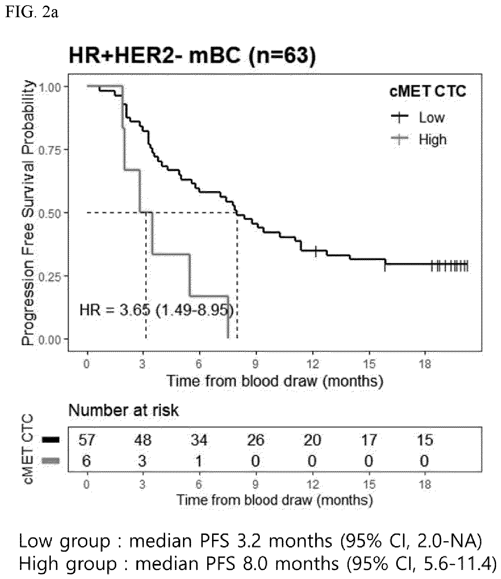

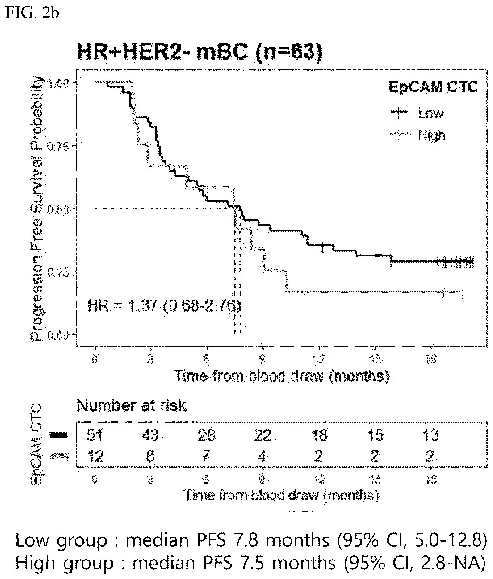

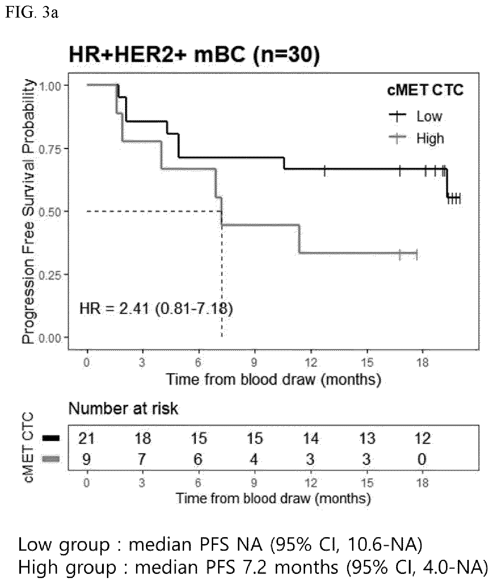

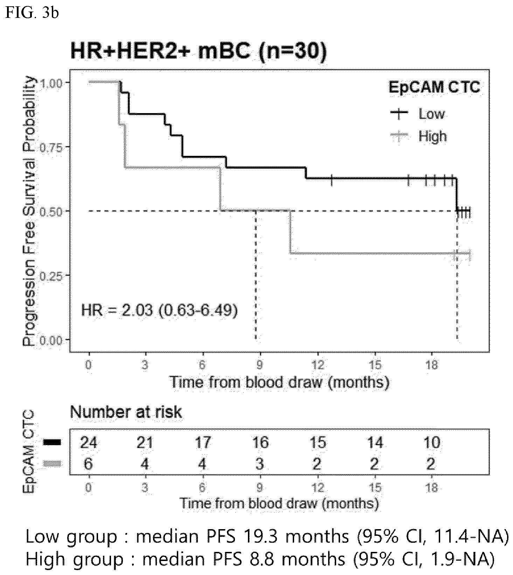

Furthermore, as shown in Figures 2a and 2b and Figures 3a and 3b, the count of c-Met-expressing CTCs was significantly associated with progression-free survival in HR+/HER2-metastatic breast cancer patients (Figure 2a) and HR+/HER2+ metastatic breast cancer patients (Figure 3a).

Additionally, univariate and multivariate analyses of clinical and pathological information and CTC count results using the Cox proportional hazards model confirmed that c-Met-expressing CTCs were statistically significant predictors of progression-free survival in HR+ breast cancer patients (see Table 2).

**Example 3. Survival Analysis Based on cfDNA Concentration**

Blood collection and analysis were conducted on the same patients as in Example 1-1. Blood was collected using a cell-free DNA blood collection tube (Streck, La Viasta, NE, USA), and patient characteristics and tumor histological details were extracted from the pathology reports of the Department of Pathology at Samsung Medical Center (SMC) (Seoul, South Korea). Disease progression was evaluated using radiological images according to the RECIST version 1.1 guidelines. Progression-free survival (PFS) was defined as the period from the time of blood collection to radiological disease progression or death from any cause.

Blood samples were centrifuged at 3000x g for 15 minutes at 4°C to separate plasma, and cfDNA was extracted from 4-6 mL plasma samples using the QIAamp Circulating Nucleic Acid Kit (Qiagen, Hilden, Germany) according to the manufacturer's protocol. cfDNA was eluted in 100 μL elution buffer. ESR1 and PIK3CA mutations in cfDNA samples were analyzed using Droplet PIK3CA and Droplex ESR1 Mutation Test Kits (Gencurix Inc., Seoul, South Korea) according to the manufacturer's protocol. ddPCR reagents were mixed with 12.9 μL of eluted cfDNA per well in 8-strip PCR tubes. Primer and probe sets were designed to detect PIK3CA hotspot mutations (R88Q, N345K, E542, E545, Q546, E726, H1047, M1048, G1049) and ESR1 hotspot mutations (E380, S463, V534, L536, Y537, D538). Positive and negative controls were mixed with the reaction mixture in 8-strip PCR tubes, and the mixture was converted to droplets using the QX200TM Droplet Generator (Bio-Rad, Hercules, CA, USA). PCR was performed on these droplets in a 96-well plate. After amplification, droplets were counted using the QX200TM Droplet Reader (Bio-Rad). Internal controls designed to detect PIK3CA or ESR1 were used as indicators of cfDNA concentration and mutation index.

cfDNA concentration and mutation index were calculated using the following formulas:

$$\text{cfDNA concentrations/mL plasma}$$

$$= \text{IC copies/well} \times \frac{\text{total DNA elution volume}}{\text{DNA loading volume/well}} \times \frac{1}{\text{plasma volume (mL)}}$$

$$\text{Mutation Index} = \frac{\text{Mutant copies of PIK3CA or ESR1}}{\text{Total copies of PIK3CA or ESR1}} \times 100\%$$

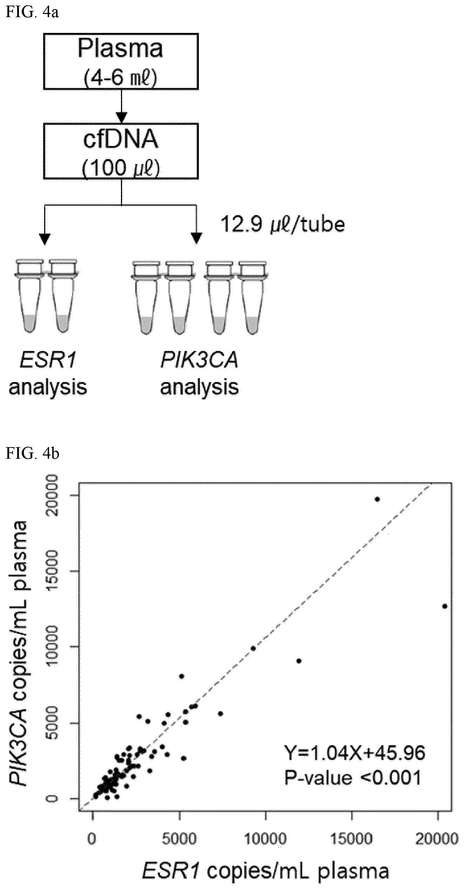

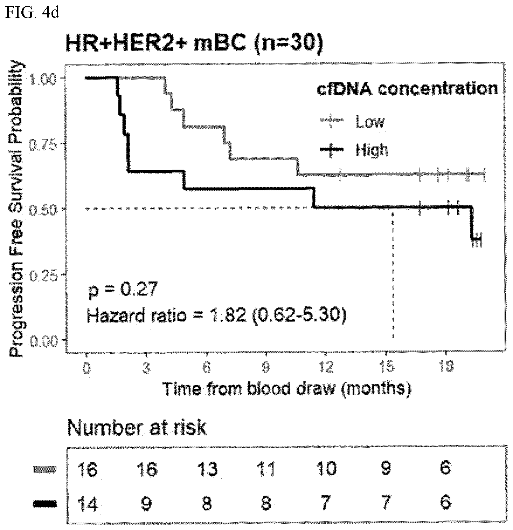

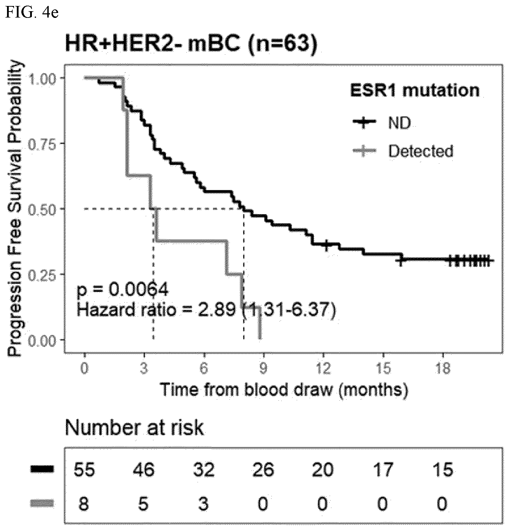

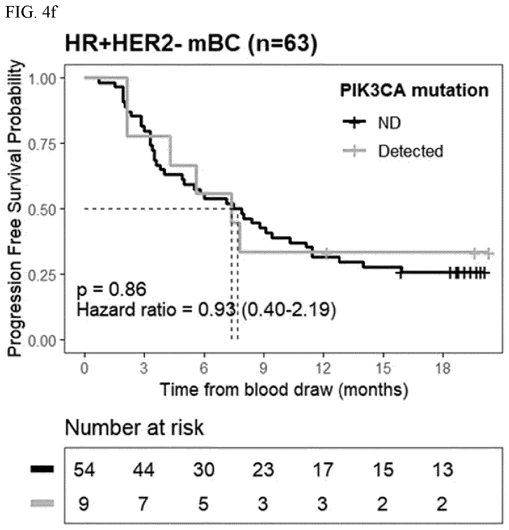

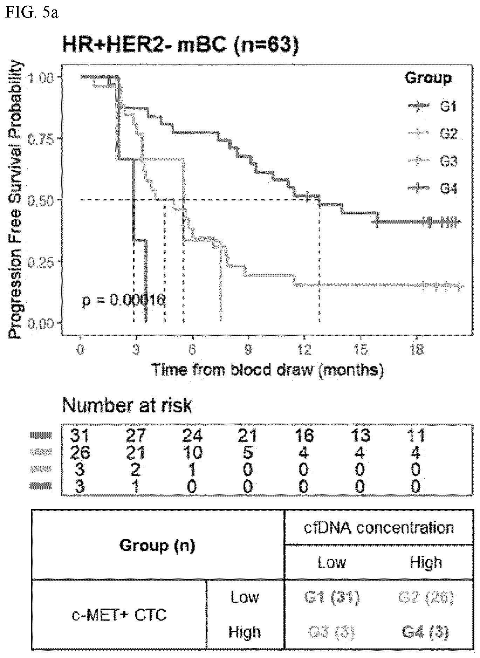

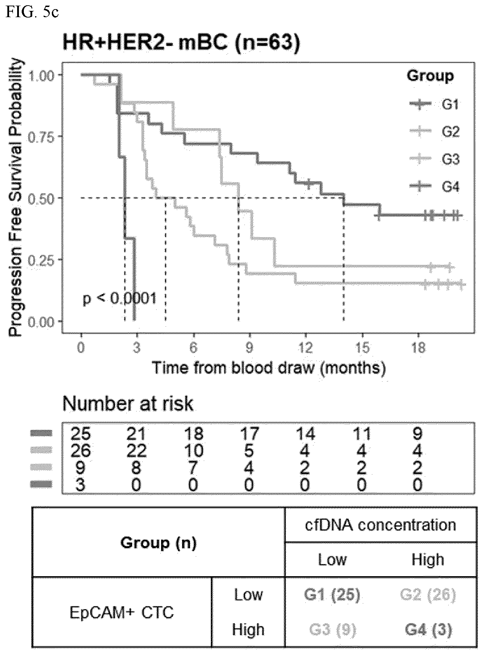

The cfDNA concentration was calculated using the IC copy number of the Droplex ESR1 Mutation Test Kits. Patients were classified as having high or low cfDNA concentration based on a cut-off value of 1490 copies/mL, which was found to have the highest statistical significance for PFS in HR+ mBC. The results are shown in Figures 4a to 4f and Figures 5a to 5d.

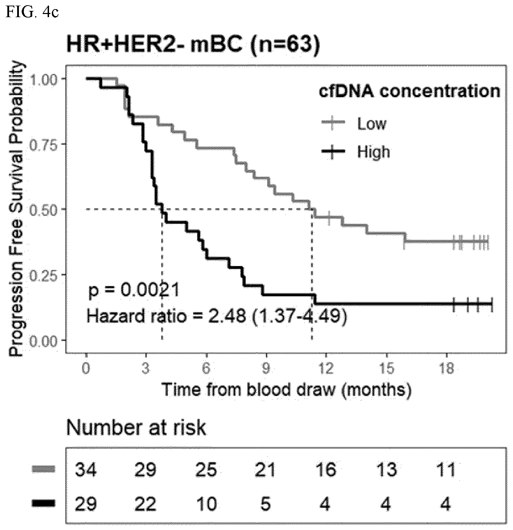

Figures 4a to 4f show the results of dividing patients into groups with high and low cfDNA concentrations, confirming that patients with high cfDNA concentrations had poorer prognosis in the HR+/HER2- mBC patient group (see Figure 4c).

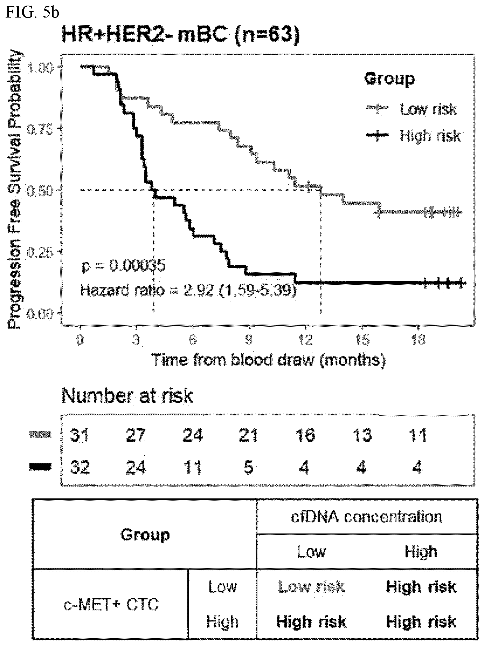

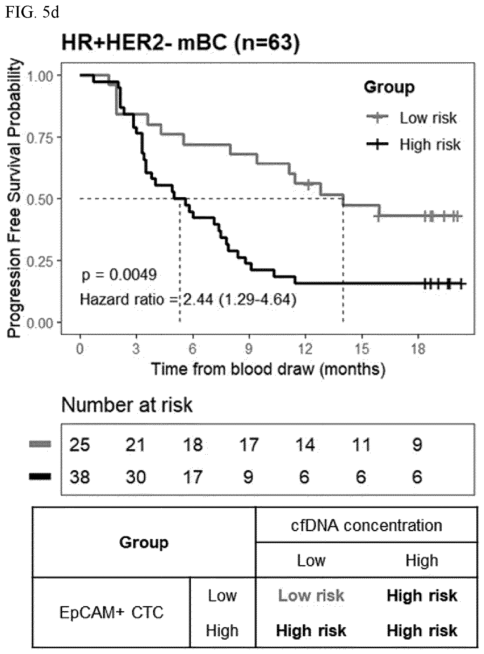

Additionally, Figures 5a to 5d show the results of dividing HR+/HER2- mBC patients into groups based on c-Met-expressing CTCs and cfDNA concentration, confirming a statistically significant difference in prognosis between patients with low levels of both c-Met-expressing CTCs and cfDNA and those with higher levels (see Figure 5b). Similar results were obtained when analyzing groups based on EpCAM-expressing CTCs and cfDNA concentration (see Figure 5d).

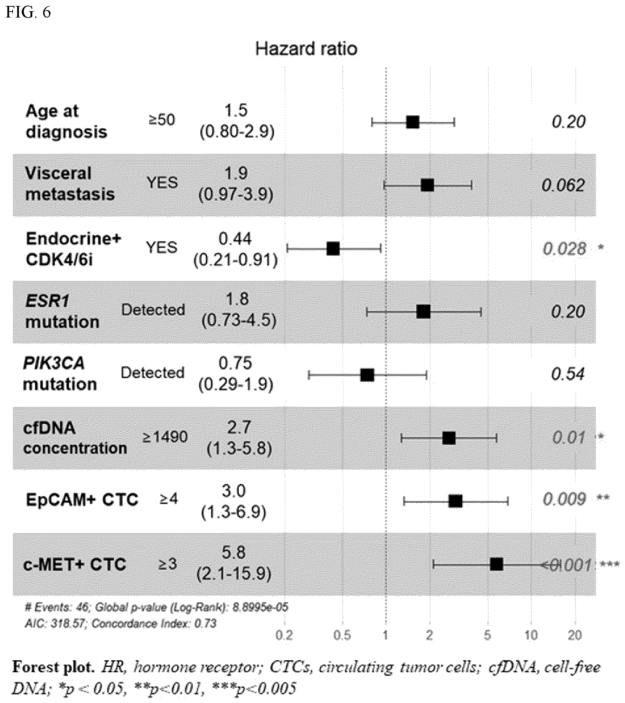

Based on these results, multivariate analysis using Cox regression confirmed that c-Met-expressing CTCs, cfDNA concentration, and EpCAM-expressing CTCs were independent factors for predicting the prognosis of breast cancer in HR+/HER2- mBC patients (see Figure 6).

**INDUSTRIAL APPLICABILITY**

As described above, the present invention provides a method for predicting the prognosis of breast cancer using c-Met enriched CTCs and a composition for predicting the prognosis of breast cancer comprising an agent that specifically binds to c-Met enriched CTCs. The method and composition of the present invention can be usefully employed to accurately predict the prognosis of breast cancer patients without the need for a tissue biopsy.

**Claims**

1.  A method for providing information necessary for predicting the prognosis of a breast cancer patient, comprising the steps of:

    (a) isolating c-Met enriched circulating tumor cells (CTCs) from a biological sample obtained from a breast cancer patient;
    (b) counting the CTCs isolated in step (a); and
    (c) predicting the prognosis of breast cancer based on the count obtained in step (b), wherein a lower count indicates a better prognosis.

2.  The method of claim 1, wherein the breast cancer is HR+/HER2- or HR+/HER2+ breast cancer.

3.  The method of claim 1, wherein the breast cancer prognosis is disease-free survival, distant metastasis-free survival, or progression-free survival.

4.  The method of claim 1, wherein the biological sample is selected from the group consisting of blood, plasma, serum, whole blood, isolated blood cells, bone marrow fluid, lymph fluid, saliva, urine, mucosal fluid, amniotic fluid, or a combination thereof.

5.  The method of claim 1, wherein the step of isolating c-Met enriched CTCs comprises using an antibody that specifically binds to the CTCs and magnetic beads that bind to the antibody.

6.  A method for providing information necessary for predicting the prognosis of a breast cancer patient, comprising the steps of:

    (a) isolating cell-free DNA (cfDNA) from a biological sample obtained from a breast cancer patient;
    (b) calculating the concentration of cfDNA or the mutation index of the ESR1 gene in the cfDNA isolated in step (a); and
    (c) predicting the prognosis of breast cancer based on the value calculated in step (b), wherein a lower value indicates a better prognosis.

7. A composition for predicting the prognosis of breast cancer, comprising an agent that specifically binds to c-Met enriched circulating tumor cells (CTCs).

8. The composition of claim 7, wherein the agent is selected from the group consisting of an antibody, aptamer, DNA, RNA, protein, or polypeptide.

9. The composition of claim 7, wherein the antibody comprises a light chain variable region (VL) including a complementarity-determining region (CDR) L1 comprising the amino acid sequence of SEQ ID NO: 1, a CDR L2 comprising the amino acid sequence of SEQ ID NO: 2, and a CDR L3 comprising the amino acid sequence of SEQ ID NO: 3, and a heavy chain variable region (VH) including a CDR H1 comprising the amino acid sequence of SEQ ID NO: 4, a CDR H2 comprising the amino acid sequence of SEQ ID NO: 5, and a CDR H3 comprising the amino acid sequence of SEQ ID NO: 6, and
wherein the antibody or a fragment thereof specifically binds to the c-Met protein.

10. The composition of claim 7, wherein the antibody comprises a light chain variable region (VL) including a complementarity-determining region (CDR) L1 comprising the amino acid sequence of SEQ ID NO: 7, a CDR L2 comprising the amino acid sequence of SEQ ID NO: 8, and a CDR L3 comprising the amino acid sequence of SEQ ID NO: 9, and a heavy chain variable region (VH) including a CDR H1 comprising the amino acid sequence of SEQ ID NO: 10, a CDR H2 comprising the amino acid sequence of SEQ ID NO: 11, and a CDR H3 comprising the amino acid sequence of SEQ ID NO: 12, and
wherein the antibody or a fragment thereof specifically binds to the c-Met protein.

11. The composition of claim 9 or claim 10, wherein the fragment is selected from the group consisting of diabody, Fab, Fab', F(ab)2, F(ab')2, Fv, and scFv.

12. Use of an agent that specifically binds to c-Met enriched circulating tumor cells (CTCs) for the manufacture of a composition for predicting the prognosis of breast cancer.

FIG. 1a

**HR+ mBC (n=93)**

Low group : median PFS 5.5 months (95% CI, 2.8-NA)
High group : median PFS 9.9 months (95% CI, 7.4-NA)

FIG. 1b

## HR+ mBC (n=93)

Low group : median PFS 9.4 months (95% CI, 5.8-NA)
High group : median PFS 7.5 months (95% CI, 2.8-NA)

FIG. 2a

**HR+HER2- mBC (n=63)**

Low group : median PFS 3.2 months (95% CI, 2.0-NA)
High group : median PFS 8.0 months (95% CI, 5.6-11.4)

FIG. 2b

**HR+HER2- mBC (n=63)**

Low group : median PFS 7.8 months (95% CI, 5.0-12.8)
High group : median PFS 7.5 months (95% CI, 2.8-NA)

FIG. 3a

HR+HER2+ mBC (n=30)

Low group : median PFS NA (95% CI, 10.6-NA)
High group : median PFS 7.2 months (95% CI, 4.0-NA)

FIG. 3b

**HR+HER2+ mBC (n=30)**

Low group : median PFS 19.3 months (95% CI, 11.4-NA)
High group : median PFS 8.8 months (95% CI, 1.9-NA)

FIG. 4a

FIG. 4b

FIG. 4c

**HR+HER2- mBC (n=63)**

p = 0.0021
Hazard ratio = 2.48 (1.37-4.49)

cfDNA concentration
— Low
— High

Number at risk

| | | | | | | |
|---|---|---|---|---|---|---|
| 34 | 29 | 25 | 21 | 16 | 13 | 11 |
| 29 | 22 | 10 | 5 | 4 | 4 | 4 |

FIG. 4d

HR+HER2+ mBC (n=30)

p = 0.27
Hazard ratio = 1.82 (0.62-5.30)

cfDNA concentration
— Low
— High

Time from blood draw (months)

Number at risk

| | | | | | | |
|---|---|---|---|---|---|---|
| 16 | 16 | 13 | 11 | 10 | 9 | 6 |
| 14 | 9 | 8 | 8 | 7 | 7 | 6 |

FIG. 4e

## HR+HER2- mBC (n=63)

p = 0.0064
Hazard ratio = 2.89 (1.31-6.37)

ESR1 mutation
— ND
— Detected

Number at risk

| | | | | | | | |
|---|---|---|---|---|---|---|---|
| ■ | 55 | 46 | 32 | 26 | 20 | 17 | 15 |
| ■ | 8 | 5 | 3 | 0 | 0 | 0 | 0 |

FIG. 4f

**HR+HER2- mBC (n=63)**

p = 0.86
Hazard ratio = 0.93 (0.40-2.19)

Number at risk

| | | | | | | | |
|---|---|---|---|---|---|---|---|
| ▬ | 54 | 44 | 30 | 23 | 17 | 15 | 13 |
| ▬ | 9 | 7 | 5 | 3 | 3 | 2 | 2 |

FIG. 5a

**HR+HER2- mBC (n=63)**

Number at risk

| | | | | | | |
|---|---|---|---|---|---|---|
| 31 | 27 | 24 | 21 | 16 | 13 | 11 |
| 26 | 21 | 10 | 5 | 4 | 4 | 4 |
| 3 | 2 | 1 | 0 | 0 | 0 | 0 |
| 3 | 1 | 0 | 0 | 0 | 0 | 0 |

| Group (n) | | cfDNA concentration | |
|---|---|---|---|
| | | Low | High |
| c-MET+ CTC | Low | G1 (31) | G2 (26) |
| | High | G3 (3) | G4 (3) |

FIG. 5b

**HR+HER2- mBC (n=63)**

Progression Free Survival Probability vs Time from blood draw (months)

Group: Low risk, High risk

p = 0.00035
Hazard ratio = 2.92 (1.59-5.39)

Number at risk

| | | | | | | | |
|---|---|---|---|---|---|---|---|
| (Low risk) | 31 | 27 | 24 | 21 | 16 | 13 | 11 |
| (High risk) | 32 | 24 | 11 | 5 | 4 | 4 | 4 |

| Group | | cfDNA concentration | |
|---|---|---|---|
| | | Low | High |
| c-MET+ CTC | Low | Low risk | High risk |
| | High | High risk | High risk |

FIG. 5c

**HR+HER2- mBC (n=63)**

Number at risk

| | | | | | | |
|---|---|---|---|---|---|---|
| 25 | 21 | 18 | 17 | 14 | 11 | 9 |
| 26 | 22 | 10 | 5 | 4 | 4 | 4 |
| 9 | 8 | 7 | 4 | 2 | 2 | 2 |
| 3 | 0 | 0 | 0 | 0 | 0 | 0 |

| Group (n) | | cfDNA concentration | |
|---|---|---|---|
| | | Low | High |
| EpCAM+ CTC | Low | G1 (25) | G2 (26) |
| | High | G3 (9) | G4 (3) |

FIG. 5d

## HR+HER2- mBC (n=63)

**Group**
- Low risk
- High risk

p = 0.0049
Hazard ratio = 2.44 (1.29-4.64)

Number at risk

| | | | | | | |
|---|---|---|---|---|---|---|
| 25 | 21 | 18 | 17 | 14 | 11 | 9 |
| 38 | 30 | 17 | 9 | 6 | 6 | 6 |

| Group | | cfDNA concentration | |
|---|---|---|---|
| | | Low | High |
| EpCAM+ CTC | Low | Low risk | High risk |
| | High | High risk | High risk |

FIG. 6

Hazard ratio

| | | | | |
|---|---|---|---|---|
| **Age at diagnosis** | ≥50 | 1.5 (0.80-2.9) | | *0.20* |
| **Visceral metastasis** | YES | 1.9 (0.97-3.9) | | *0.062* |
| **Endocrine+ CDK4/6i** | YES | 0.44 (0.21-0.91) | | *0.028 \** |
| *ESR1* **mutation** | Detected | 1.8 (0.73-4.5) | | *0.20* |
| *PIK3CA* **mutation** | Detected | 0.75 (0.29-1.9) | | *0.54* |
| **cfDNA concentration** | ≥1490 | 2.7 (1.3-5.8) | | *0.01 \** |
| **EpCAM+ CTC** | ≥4 | 3.0 (1.3-6.9) | | *0.009 \*\** |
| **c-MET+ CTC** | ≥3 | 5.8 (2.1-15.9) | | *<0.001 \*\*\** |

*# Events: 46; Global p-value (Log-Rank): 8.8995e-05*
*AIC: 318.57; Concordance Index: 0.73*

0.2    0.5    1    2    5    10    20

**Forest plot.** *HR, hormone receptor; CTCs, circulating tumor cells; cfDNA, cell-free DNA; \*p < 0.05, \*\*p<0.01, \*\*\*p<0.005*

## INTERNATIONAL SEARCH REPORT

| International application No. |
|---|
| **PCT/KR2023/005374** |

### A. CLASSIFICATION OF SUBJECT MATTER
**G01N 33/574**(2006.01)i; **C07K 16/28**(2006.01)i

According to International Patent Classification (IPC) or to both national classification and IPC

### B. FIELDS SEARCHED

Minimum documentation searched (classification system followed by classification symbols)

G01N 33/574(2006.01); C07K 16/28(2006.01); C12N 5/077(2010.01); C12N 5/0775(2010.01); C12N 5/09(2010.01); C12Q 1/6886(2018.01); G01N 33/543(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 순환종양세포(circulating tumor cell), c-Met, 유방암(breast cancer), 예후(prognosis), cfDNA(cell-free DNA), 유전자 돌연변이(gene mutation)

### C. DOCUMENTS CONSIDERED TO BE RELEVANT

| Category* | Citation of document, with indication, where appropriate, of the relevant passages | Relevant to claim No. |
|---|---|---|
| X | KR 10-2019-0038173 A (SEOUL NATIONAL UNIVERSITY R&DB FOUNDATION et al.) 08 April 2019 (2019-04-08)<br>See claims 1, 2 and 7-13; and paragraphs [0005] and [0223]-[0228]. | 1-5,7,8,11,12 |
| Y | | 9,10 |
| A | | 6 |
| X | KR 10-2021-0112118 A (CLINOMICS INC.) 14 September 2021 (2021-09-14)<br>See claims 1, 3, 4, 7-11, 15 and 16. | 6 |
| DY | KR 10-2020-0069822 A (SEOUL NATIONAL UNIVERSITY R&DB FOUNDATION et al.) 17 June 2020 (2020-06-17)<br>See claim 1; and SEQ ID NOs: 1 to 6. | 9 |

| ✓ | Further documents are listed in the continuation of Box C. | ✓ | See patent family annex. |
|---|---|---|---|

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **07 August 2023** | **07 August 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| | International application No. |
|---|---|
| | **PCT/KR2023/005374** |

### C. DOCUMENTS CONSIDERED TO BE RELEVANT

| Category* | Citation of document, with indication, where appropriate, of the relevant passages | Relevant to claim No. |
|---|---|---|
| DY | KR 10-2020-0069839 A (SEOUL NATIONAL UNIVERSITY R&DB FOUNDATION et al.) 17 June 2020 (2020-06-17)<br>    See claim 1; and SEQ ID NOs: 1 to 6. | 10 |
| X | KR 10-2019-0038174 A (SEOUL NATIONAL UNIVERSITY R&DB FOUNDATION et al.) 08 April 2019 (2019-04-08)<br>    See claims 1, 2 and 7-13; and paragraphs [0005] and [0223]-[0228]. | 1-5,7,8,11,12 |
| A | ZHANG, T. et al. Development of Novel c-MET-Based CTC Detection Platform. Molecular Cancer Research. 2016, vol. 14, no. 6, pp. 539-547.<br>    See entire document. | 1-12 |

Form PCT/ISA/210 (second sheet) (July 2022)

<table>
<tr><td>**INTERNATIONAL SEARCH REPORT**</td><td>International application No.<br>**PCT/KR2023/005374**</td></tr>
</table>

**Box No. I    Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

       ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
Information on patent family members

International application No.

**PCT/KR2023/005374**

| Patent document cited in search report | | | Publication date (day/month/year) | Patent family member(s) | | | Publication date (day/month/year) |
|---|---|---|---|---|---|---|---|
| KR | 10-2019-0038173 | A | 08 April 2019 | WO | 2019-066617 | A2 | 04 April 2019 |
| | | | | WO | 2019-066617 | A3 | 04 July 2019 |
| | | | | WO | 2019-066617 | A9 | 15 August 2019 |
| KR | 10-2021-0112118 | A | 14 September 2021 | KR | 10-2422776 | B1 | 21 July 2022 |
| KR | 10-2020-0069822 | A | 17 June 2020 | AU | 2019-391657 | A1 | 01 July 2021 |
| | | | | AU | 2019-391657 | B2 | 22 September 2022 |
| | | | | AU | 2022-235579 | A1 | 13 October 2022 |
| | | | | CN | 113227143 | A | 06 August 2021 |
| | | | | EP | 3892635 | A1 | 13 October 2021 |
| | | | | JP | 2022-511002 | A | 28 January 2022 |
| | | | | KR | 10-2396194 | B1 | 10 May 2022 |
| | | | | US | 2022-0220207 | A1 | 14 July 2022 |
| | | | | WO | 2020-117017 | A1 | 11 June 2020 |
| KR | 10-2020-0069839 | A | 17 June 2020 | AU | 2019-392156 | A1 | 01 July 2021 |
| | | | | AU | 2019-392156 | B2 | 22 September 2022 |
| | | | | AU | 2022-235582 | A1 | 13 October 2022 |
| | | | | CN | 113195536 | A | 30 July 2021 |
| | | | | EP | 3896087 | A1 | 20 October 2021 |
| | | | | JP | 2022-510988 | A | 28 January 2022 |
| | | | | KR | 10-2433184 | B1 | 17 August 2022 |
| | | | | US | 2022-0220208 | A1 | 14 July 2022 |
| | | | | WO | 2020-117019 | A1 | 11 June 2020 |
| KR | 10-2019-0038174 | A | 08 April 2019 | WO | 2019-066620 | A2 | 04 April 2019 |
| | | | | WO | 2019-066620 | A3 | 08 August 2019 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020220049175
- KR 1020200069822
- KR 1020200069839
- US 4816567 A
- US 5939598 A